(19) Europäisches Patentamt
European Patent Office
Office européen des brevets

(11) EP 0 792 632 B2

(12) # NOUVEAU FASCICULE DE BREVET EUROPEEN

Après la procédure d'opposition

(45) Date de publication et mention de la décision concernant l'opposition:
**16.02.2011 Bulletin 2011/07**

(45) Mention de la délivrance du brevet:
**06.05.1998 Bulletin 1998/19**

(21) Numéro de dépôt: **97400242.0**

(22) Date de dépôt: **03.02.1997**

(51) Int Cl.:
***A61K 8/04*** *(2006.01)*

(54) **Dispositif pressurisé transparent à composition moussante comprenant des tensioactifs non-ioniques et amphotères**

Unter Druck stehende transparente Vorrichtung mit schäumender Zusammensetzung die nichtionische und amphotere Oberflächen-aktive Verbindungen enthält

Pressurized transparent apparatus with a foaming composition containing non ionic and amphoteric surfactants

(84) Etats contractants désignés:
**AT BE CH DE DK ES FR GB IT LI NL SE**

(30) Priorité: **01.03.1996 FR 9602628**

(43) Date de publication de la demande:
**03.09.1997 Bulletin 1997/36**

(73) Titulaire: **L'Oréal**
**75008 Paris (FR)**

(72) Inventeur: **Maurin, Véronique**
**75016 Paris (FR)**

(74) Mandataire: **Dossmann, Gérard**
**Casalonga & Partners**
**Bayerstrasse 71-73**
**80335 München (DE)**

(56) Documents cités:

| | |
|---|---|
| EP-A- 0 194 097 | EP-A- 0 213 827 |
| EP-A- 0 304 713 | EP-A- 0 577 506 |
| EP-A- 0 586 295 | WO-A-93/09761 |
| WO-A-95/05796 | WO-A1-96/32921 |
| AU-A- 627 666 | CA-A- 1 002 256 |
| DE-A1- 4 327 699 | FR-A1- 2 644 173 |
| GB-A- 1 121 563 | US-A- 4 627 973 |
| US-A- 5 156 833 | US-A- 5 167 950 |
| US-A- 5 429 815 | |

- **PATENT ABSTRACTS OF JAPAN vol. 12, no. 467 (C-550) [3314] , 7 Décembre 1988 & JP 63 185911 A (KANEBO LTD), 1 Août 1988,**
- **PATENT ABSTRACTS OF JAPAN vol. 13, no. 339 (C-624) [3687] , 31 Juillet 1989 & JP 01 117817 A (POLA CHEM ND INC)**

EP 0 792 632 B2

## Description

L'invention a pour objet de nouveaux dispositifs pressurisés comprenant une composition, notamment une composition cosmétique ou dermatologique, et en particulier une composition nettoyante et démaquillante.

L'utilisateur attend d'une composition démaquillante qu'elle permette d'ôter toutes sortes de produits de maquillage: rouge à lèvres, poudre, ombre à paupières, fond de teint etc..., sans abimer la peau et en la laissant propre et douce. Le nettoyage de la peau est très important pour le soin du visage. Il doit être le plus performant possible car les résidus gras tels que l'excès de sébum, les restes des produits cosmétiques utilisés quotidiennement, notamment les produits de maquillage, et en particulier les produits « waterproof » résistants à l'eau, s'accumulent dans les replis cutanés et peuvent obstruer les pores de la peau et entraîner l'apparition de boutons. Les démaquillants habituels sont généralement sous la forme d'un gel, d'une lotion ou d'une crème. Toutefois, le marché attend de nouvelles formes de produits, à la fois plus esthétiques et éventuellement ludiques. Pour répondre à cette attente, de nouveaux démaquillants et nettoyants pour la peau sont apparus, sous la forme de dispositifs pressurisés transparents, contenant une composition nettoyante et démaquillante transparente, monophasique et moussante.

On connait, en particulier par le brevet américain US 3,719,752, des compositions pour application topique, conditionnées en bouteilles pressurisées, moussantes, transparentes, monophasiques et stables. Ces compositions comprennent un tensioactif et un gaz propulseur dans un milieu hydroalcoolique. L'alcool est de préférence un alcool monohydrique en $C_1$-$C_3$. Toutefois l'emploi d'alcool, et en particulier d'éthanol, dans de telles compositions implique un risque lié à l'inflammabilité. On a par conséquent cherché à éviter l'emploi d'alcool monohydrique en $C_1$-$C_3$ dans les compositions nettoyantes pour le visage.

Par ailleurs, le document WO95/05796 décrit un dispositif pressurisé comprenant une composition monophasique, fluide, exempte d'alcool monohydrique, constituée d'un gaz propulseur hydrocarboné et d'un concentré aqueux comprenant des sels d'$\alpha$-oléfine sulfonate, des bétaines et des alkylpolyglucosides, contenue dans une bouteille transparente. Toutefois ce dispositif n'est pas dépourvu d'inconvénients : la composition qu'il renferme n'est pas stable dans le temps ; en particulier on voit apparaître au stockage un halo d'agrégats en suspension dans la composition. La bouteille pressurisée étant transparente, une telle instabilité n'est pas acceptable pour le consommateur

Le document EP-676188 révèle un dispositif analogue, comprenant une composition monophasique, fluide, exempte d'alcool monohydrique, constituée d'un gaz propulseur et d'un concentré aqueux. Le gaz propulseur comprend un mélange de diméthyléther et de gaz hydrocarboné; le concentré aqueux comprend des tensioactifs et en outre un agent de couplage. On sait par ailleurs que le diméthyléther est un gaz inflammable, de très bas point d'éclair Son utilisation accroît donc les risques liés aux dispositifs pressurisés, aussi bien pour le site de production que pour l'utilisateur de ce dispositif.

Le brevet canadien CA-1002256 révèle un système pressurisé transparente, uniforme utilisable pour la distribution de produits cosmétiques aqueux transparents, dont des produits démaquillants. L'utilisation comme tensioactif d'un sel de triéthanolamine d'un acide acyl lactylique permet d'obtenir un système dans lequel le gaz propulseur est complètement dissout dans la phase aqueuse.

Le brevet américain US 3,840,465 révèle un système pressurisé transparent comprenant une composition transparente moussante, exempte d'alcool. Cette composition comprend une solution aqueuse d'un tensioactif moussant, qui est généralement anionique, dans laquelle est dispersé un propulseur liquide grâce à un second tensioactif, soluble dans le propulseur et insoluble dans l'eau.

Les compositions décrites dans l'ensemble de ces documents et utilisées pour le nettoyage de la peau sont essentiellement constituées de tensioactifs anioniques, qui détruisent le film hydro-lipidique de la peau et laissent la peau propre mais rêche.

Aussi, c'est avec étonnement que la demanderesse a découvert de nouveaux dispositifs comprenant une composition cosmétique transparente, stable, moussante, très douce, pressurisée par un gaz hydrocarboné exempte d'alcool monohydrique en $C_1$-$C_3$, à base de tensioactifs non-ioniques et comprenant éventuellement un ou plusieurs tensioactifs amphotères.

L'invention a pour objet un dispositif pressurisé comprenant

(i) une bouteille pressurisée munie d'une tête de distribution,
(ii) une composition transparente monophasique contenue dans la bouteille et constituée par

A- 0,5 à 10% d'un gaz propulseur, choisi parmi les gaz hydrocarbonés ou fluorés
B-90 à 99,5% d'une composition aqueuse, exempte d'alcools monohydriques en $C_1$-$C_3$

ce dispositif étant apte à distribuer une mousse lorsque l'on actionne la tête de distribution, caractérisé en ce que la composition B comprend un mélange de tensioactifs choisis parmi les tensioactifs non-ioniques et les tensioactifs amphotères, en l'absence de tensioactifs anioniques et antioniques.

Lorsque l'on actionne le bouton-poussoir faisant partie de la tête de distribution, les compositions de l'invention sont distribuées sous la forme d'une mousse crémeuse qui nettoie la peau sans l'agresser L'absence de tensioactifs anioniques et cationiques dans les compositions distribuées par ce dispositif a pour effet l'obtention d'une mousse d'un produit extrêmement doux pour la peau et pour les muqueuses (lèvres, intérieur des paupières), ce qui permet son utilisation pour le nettoyage et le démaquillage du visage, en particulier le démaquillage des yeux.

De façon préférentielle, les bouteilles pressurisées utilisées dans l'invention sont transparentes. De telles bouteilles sont bien connues de l'homme du métier et d'utilisation courante, on pourra par exemple se référer à la demande de brevet WO95/05796 au sujet de ces bouteilles. Elles mettent particulièrement en valeur le caractère surprenant de l'invention : au travers de la bouteille on observe une émulsion transparentes, ayant une fluidité comparable à celle de l'eau, ladite émulsion étant restituée sous la forme d'une mousse onctueuse au travers du moyen de distribution, et non sous la forme d'un spray comme on s'y attend à priori. On peut utiliser n'importe quel moyen de distribution de bouteille aérosol connu de l'homme du métier. Une bouteille et un moyen de distribution pouvant être utilisés dans la présente invention est par exemple représenté dans la demande de brevet FR-95-12788.

Le mot «transparent» signifie qu'au travers de la bouteille contenant la composition on peut distinguer les caractères imprimés sur une page de journal placée derrière cette bouteille. Ce terme signifie également qu'un échantillon de 10cm d'épaisseur de la composition a une transmission maximum de la lumière d'au moins 4% dans n'importe quelle longueur d'onde comprise entre 200nm et 800nm.

Plus particulièrement, l'invention a pour objet un dispositif pressurisé tel que décrit ci-dessus caractérisé en ce que la composition aqueuse B comprend :

(a) au moins un tensio-actif émulsionnant non-ionique,
(b) au moins un tensioactif épaississant non-ionique.

De préférence, la composition aqueuse B comprend 1 à 25% en poids d'au moins un tensioactif émulsionnant non-ionique.

De façon avantageuse, la composition aqueuse B comprend 1 à 30% d'au moins un tensioactif épaississant non-ionique.

De préférence, la composition de B est ajustée, et en particulier le choix et le pourcentage du ou des épaississants, de telle sorte que cette composition B a une viscosité Brookfield comprise entre 5 et 200mPa.s, et de préférence entre 10 et 150mPa.s.

On peut citer en particulier les gaz hydrocarbonés, comme par exemple le propane, le n-butane, l'isobutane et leurs mélanges ; les gaz fluorés comme par exemple le chlorodifluoro méthane, le dichlorodifluorométhane, le difluoroéthane, le chlorodifluoroéthane, le dichlorotétrafluoroéthane etc... et leurs mélanges. . De façon préférentielle, on utilise dans la présente invention des gaz hydrocarbonés ayant de 2 à 6 atomes de carbone. Parmi les compositions de l'invention, on préfère celles ayant de 1,5 à 4%, en poids par rapport au poids total de la composition, de gaz propulseur.

Les tensioactifs émulsionnants utilisables dans la présente invention sont préférentiellement choisis parmi ceux ayant une balance HLB comprise entre 12 et 18. On calcule la balance HLB (balance hydrophile-lipophile) d'un émulsionnant suivant la formule suivante :

$$HLB = \frac{100-L}{5}$$

dans laquelle L représente le pourcentage en poids du groupement lipophile par rapport au poids de la molécule entière.

Préférentiellement, ces tensioactifs sont choisis parmi les produits d'addition de 1 à 200 moles d'oxyde d'éthylène ou d'oxyde de propylène sur des esters partiels de polyols ayant 2 à 16 atomes de carbone et d'acides gras ayant 12 à 22 atomes de carbone. Avantageusement, on choisit des produits tels que décrits ci-dessus ayant un taux d'estérification inférieur ou égal à 2.

Les tensioactifs épaississants non-ioniques utilisables dans la présente invention sont préférentiellement choisis parmi :

$\alpha$-les $C_1$-$C_6$ alcanolamides d'acides $C_8$-$C_{22}$ alkyl éther carboxyliques ;
$\beta$- les produits d'addition de 10 à 300 moles d'oxyde d'éthylène ou d'oxyde de propylène sur des esters partiels de polyols ayant 2 à 16 atomes de carbone et d'acides gras ayant 12 à 22 atomes de carbone. Parmi cette famille de produits, on choisit préférentiellement ceux ayant un taux d'estérification supérieur ou égal à 2 ;
$\gamma$- les alcools gras en $C_{12}$-$C_{22}$ polyoxyéthylénés et/ou polyoxypropylénés et/ou polyglycérolés,
$\delta$- les esters gras en $C_{12}$-$C_{22}$ de polyoxyéthylène et/ou de polyoxypropylène et/ou de polyglycérol,
$\varepsilon$- les polymères blocs de polyéthylène glycol et/ou de polypropylène glycol.

Avantageusement, les compositions selon l'invention comprennent au moins deux tensioactifs épaississants appartenant chacun à deux familles différentes, parmi les familles d'épaississants $\alpha$ à $\varepsilon$ décrites ci-dessus.

Par $C_1$-$C_6$ alcanolamide d'acide $C_8$-$C_{22}$ alkyl éther carboxylique, on désigne des produits répondant à la formule 1:

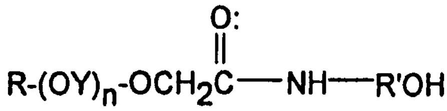

(I)

dans laquelle R représente une chaîne alkyle ayant 8 à 30 atomes de carbone, R' représente une chaîne alkyle ayant 1 à 6 atomes de carbone, Y représente un radical éthylène di-yle ou propylène 1,3-di-yle et n représente un nombre entier allant de 0 à 100.

Par exemple, R peut être choisi parmi les radicaux stéaryle, oléyle, ricinoléyle, linoléyle, lauryle, myristyle, capryle, palmityle ou parmi des mélanges de radicaux alkyles en $C_8$-$C_{22}$.

R' peut par exemple être choisi parmi les radicaux méthyle, éthyle, n- propyle, isopropylé, terbutyle, n-hexyle.

Parmi les esters partiels de polyols utilisables dans la présente invention on peut citer particulièrement ceux qui dérivent du glycol, du glycérol et des sucres, comme par exemple du glucose, du fructose, du maltose, du ribose, de l'arabinose, du xylose, du lyxose, du ribulose, du xylulose, de l'allose, de l'altrose, du mannose, du gulose, du galactose, du saccharose, du sorbitol.

Parmi les tensioactifs épaississants utilisables dans la présente invention on peut citer par exemple les produits suivants: l'huile de ricin, les monoéthanolamides d'acide alkyl ($C_{13}$, $C_{15}$) éther carboxylique (2 OE), la monoéthanolamide d'acide éther carboxylique (50 OE), les mono- et di-glycérides de suif oxyéthylénés (200 OE), les mono- et di-glycérides de suif oxyéthylénés (82 OE), les mono- et di-glycérides de coprah oxyéthylénés (78 OE), les triglycérides d'acide stéarique et hydroxystéarique (40 OE), le mono-stéarate de polyéthylène glycol (100 OE), l'alcool dodécanediol polyglycérolé (3,5moles) et de façon plus générale les produits décrits dans le brevet FR-2091516, l'octyl-2 dodécanol oxyéthyléné (250 OE), l'alcool stéarylique oxyéthyléné (100 OE), l'alcool oléique oxyéthyléné (50 OE), le di-otéato de polyéthylène glycol (50 OE), le palmitate de glycérol oxyéthyléné (200 OE), le tri-isostéarate de sorbitane oxyéthyléné (160 OE), le tri-isostéarate de sorbitane oxyéthyléné (200 OE), le stéarate de polyéthylèneglycol (150 OE), le di-oléate de méthyl glucose oxyéthyléné (120 OE), le tétra-stéarate de penta-érythrityle oxyéthyléné (150 OE), le di-oléate de polyéthylène glycol (55 OE), l'undécylénate de polyéthylène glycol (300 OE), le di-stéarate de polyéthylène glycol (150 OE),

Parmi les esters de polyols polyoxyalkylénés utilisables comme tensioactifs émulsionnants on peut citer par exemple les produits suivants: le mono-undécylénate de glycérol oxyéthyléné (7 OE), le monostéarate de glycérol oxyéthyléné (15 OE), le mono-laurate de glycérol oxyéthyléné (20 OE), le di-laurate de glycérol oxyéthyléné (20 OE), le stéarate de di-glycérol oxyéthyléné (4 OE), le cocoate de glycérol oxyéthyléné (7 OE), le pyrrolidone carboxylate de monoisostéarate de glycérol oxyéthyléné (25OE), le mono stéarate de glycérol oxyéthyléné (20 OE), le monostéarate de glycérol oxyéthyléné (30 OE), le stéarate de di-glycérol oxyéthyléné (4 OE), les mono- et di-glycérides de coprah oxyéthyléné (30 OE), le lanolate de sorbitane oxyéthyléné (40 OE), le mono-oléate de sorbitane oxyéthyléné (20 OE), le tri-oléate de sorbitane oxyéthyléné (20 OE), le mono-palmitate de sorbitane oxyéthyléné (20 OE), le mono-stéarate de sorbitane oxyéthyléné (20 OE), le mono-lau rate de sorbitane oxyéthyléné (20 OE), l'undécylénate de sorbitane oxyéthyléné (20 OE), l'undécylénate de sorbitane oxyéthyléné (18 OE), l'hexa-stéarate de sorbitane oxyéthyléné (6 OE), le monolaurate de sorbitane oxyéthyléné (44 OE), le tétra-oléate de sorbitane oxyéthyléné (30 OE), le mono-stéarate de sorbitane oxyéthyléné (4 OE), l'oléate de sorbitane oxyéthyléné (40 OE), le mono-laurate de sorbitane oxyéthyléné (4 OE), le mono-laurate de sorbitane oxyéthyléné (10 OE), le tétra-oléate de sorbitane oxyéthyléné (40 OE), le cocoate de butyl glucoside oxyéthyléné (3 OE), le di-stéarate de méthyl glucoside oxypropyléné (20 OP), le mono-laurate de méthyl glucoside oxyéthyléné (20 OE), le benzoate de méthyl glucose oxyéthyléné (20 OE), les esters de tri-glycérides de ricin et de saccharose oxyéthylénés (1,4 OE), les esters de tri-glycérides de ricin et de saccharose oxyéthylénés (2 OE), le sesquistéarate de méthyl glucose oxyéthyléné (20 OE), le di-stéarate de méthyl glucose oxyéthyléné (20 OE), le mono-stéarate de butanediol oxyéthyléné (4 OE), le mono-stéarate de polyéthylène glycol oxyéthyléné (50 OE), le di-stéarate de polyéthylène glycol, le myristate de polyéthylène glycol (8 OE), le mono-stéarate de polyéthylène e glycol (8 OE), le mono-stéarate de polyéthylène glycol (20 OE), le laurate de polyéthylène glycol (6 OE), le di-stéarate de polyéthylène glycol (8 OE), le di-laurate de polyéthylène glycol (8 OE), le mono-oléate de polyéthylène glycol (8 OE), le di-heptanoate de tétra-éthylène glycol, le di-oléate de polyéthylène glycol (8 OE), l'oléate de polypropylène glycol (26 OE), le di-polyhydroxystéarate (6 hydroxy) de polyéthylène glycol (30 OE), le stéarate de polyéthylène glycol (40 OE), le di-lautate de polyéthylène glycol (8 OE).

L'ensemble des produits utilisés dans la présente invention sont disponibles commercialement :

- les $C_1$-$C_6$ alcanolamides d'acides $C_8$-$C_{22}$ alkyl éther carboxylique sont par exemple commercialisés par la société HENKEL sous la marque COMPERLAN, ou par la société CHEM Y sous le nom de marque AMINOL;
- les produits d'addition d'oxyde d'éthylène ou d'oxyde de propylène sur des esters partiels de polyols et d'acides gras sont par exemple commercialisés par la société BASF sous la marque CREMOPHOR, ou par la société AMERCHOL sous la marque GLUCAMATE ;
- les alcools gras en $C_{12}$-$C_{22}$ polyoxyéthylénés et/ou polyoxypropylénés et/ou polyglycérolés sont par exemple commercialisés par la société ICI sous la marque BRIJ,
- les esters gras en $C_{12}$-$C_{22}$ de polyoxyéthylène et/ou de polyoxypropylène et/ou de polyglycérol sont par exemple commercialisés par la société ICI sous la marque MYRJ,
- les polymères blocs de polyéthylène glycol et/ou de polypropylène glycol sont par exemple commercialisés par la société ICI sous la marque SYNPERONIC.

De façon préférentielle, l'invention a pour objet un dispositif pressurisé tel que décrit ci-dessus caractérisé en ce que la composition aqueuse B comprend :

(a) 2 à 15% et encore plus préférentiellement de 3 à 12% en poids d'au moins un tensioactif émulsionnant non-ionique,
(b) 2 à 15% en poids d'au moins un tensioactif épaississant non-ionique,

Les compositions de la présente invention comprennent de préférence de 3 à 10% d'au moins un tensio-actif épaississant tel que défini ci-dessus. En pratique, la quantité d'épaississant utilisée est adaptée pour satisfaire le critère de viscosité donné ci-dessus. Une quantité excessive de ce type de composé peut entraîner un épaississement important de la composition de l'invention, qui devient alors difficile à distribuer en aérosol. Une quantité trop faible présente l'inconvénient de mettre en péril la stabilité de la composition.

Avantageusement les compositions B comprennent en outre au moins un alkylpolyglucoside comme tensio-actif non-ionique. De façon préférentielle, cet alkylpolyglucoside représente de 0,01 à 10% en poids de B.

De préférence, la composition aqueuse B comprend en outre au moins un tensioactif amphotère, avantageusement en quantités allant de 0,01 à 15% en poids de B et encore plus préférentiellement de 1 à 5% en poids de B.

Selon une composition préférée, B comprend :

- 1 à 25% en poids d'au moins un tensioactif émulsionnant non-ionique,
- 1 à 30% en poids d'au moins un tensioactif épaississant non-ionique,
- 0,01 à 10% en poids d'au moins un alkylpolyglucoside,
- 0,01 à 15% en poids d'au moins un tensioactif amphotère.

Les compositions B peuvent comprendre jusqu'à environ 98% d'eau. Habituellement on entend par eau de l'eau pure déminéralisée. Toutefois tout ou partie de l'eau utilisée dans les compositions B peut éventuellement être choisie parmi les eaux minérales ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

Les compositions cosmétiques ou dermatologiques de l'invention peuvent, en outre, contenir des adjuvants habituels dans les domaines concernés tels que les conservateurs, les antioxydants, les parfums, les filtres, les matières colorantes, les actifs hydrophiles ou lipophiles,.

Les actifs pour la peau peuvent être des actifs anti-âge, des actifs anti-rides, des hydratants ou des humectants, des actifs dépigmentants, des actifs anti-radicaux libres (espèces radicalaires de l'oxygène), des actifs nutritifs, des actifs protecteurs, des actifs restructurants, des actifs raffermissants, des actifs antiacnéiques, des actifs exfoliants, des actifs émollients ou encore des actifs traitants des maladies de peau comme les mycoses, les dermites, le psoriasis,

etc. Ces actifs sont utilisés, selon leur nature, dans les proportions habituelles et par exemple de 0,01 % à 10 % en poids par rapport au poids total de la composition.

Comme actifs anti-acnéiques, anti-âge, anti-ride, hydratants, exfoliants, on peut citer plus particulièrement les $\alpha$-hydroxy-acides (acides glycolique, lactique, malique, citrique...).

Comme anti-oxydants on peut citer par exemple les sulfites.

**Exemples :**

Les exemples suivants sont donnés à titre d'illustration de l'invention et n'ont pas de caractère limitatif.

Des compositions nommées $B_1$ à $B_{16}$ ont été préparées conformément aux informations données dans le tableau I. Ces compositions sont ensuite introduites dans une bouteille pressurisée munie d'une tête de distribution dans laquelle on introduit un gaz pressurisé. L'ensemble est agité de façon à obtenir un mélange homogène des deux phases initiales. Les dispositifs sont évalués dans le tableau 1.
Dans le tableau 1:

> Toutes les quantités sont données en pourcentage en poids de matière active par rapport au poids total de la composition.

Le tensio-actif amphotère N-cocoyl amidoéthyl, N-éthoxycarboxyméthyl glycinate de sodium est commercialisé sous le nom de «Miranol C2M» par la société Rhône-Poulenc, le «Plantaren 2000», commercialisé par la société Henkel, est un mélange d'alkyl ($C_8$/$C_{10}$/$C_{12}$/$C_{14}$/$C_{16}$ 29/37/23/9/2) polyglucosides, «TWEEN 20» désigne le mono-laurate de sorbitane oxyéthyléné (20 OE) (tensio-actif non-ionique) commercialisé par la société ICI. A l'exception de l'eau de Lucas, l'eau employée dans les exemples est une eau déminéralisée.

On a évalué la viscosité des compositions aqueuses $B_1$ à $B_{16}$ en mPa.s, puis on a conditionné ces compositions aqueuses dans des bouteilles pressurisées transparentes en introduisant dans ces bouteilles 97% en poids de la composition et 3% en poids d'isobutane. On a ensuite mesuré la transparence et la stabilité, après plusieurs mois de stockage à température ambiante, de ces dispositifs pressurisés.
La viscosité est donnée en mPa.s. Elle est mesurée à 25°C à l'aide d'un viscosimètre Brookfield dont la vitesse du module tournant (module 1) est fixée à 5 tours/min.

- la transparence est notée +, 0, ou-:

  += transparent, 0 = présence d'un halo, - = trouble

- la stabilité est notée 0 ou 1: 1 = stable, 0 = instable

Les différents épaississants sont désignés $E_1$ à $E_{10}$:

$E_1$ : mono éthanolamide d'acides de Coprah
$E_2$ : huile de ricin
$E_3$ : alcool oléique OE (50 OE)
$E_4$ : amide d'acide stéarique ($C_{16}$/$C_{18}$. 30/70) (50 OE)
$E_5$: di-oléate de méthyl glucoside (120 OE)
$E_6$ : Tri-isostéarate de sorbitane oxyéthyléné (160 OE)
$E_7$: alcool dodécanediol polyglycérolé (3,5 moles)
$E_8$: monostéarate de PEG (100 OE)
$E_9$: polymère séquencé de polyoxyéthylène et de polyoxypropylène (OE/OP/OE 98/67/98)
$E_{10}$ : glycéride de palme oxyéthyléné (200 OE) et glycéride de coprah oxyéthyléné (70 OE) (80/20)
$E_{11}$ : alcool cétylstéarylique ($C_{16}$/$C_{18}$) oxyéthyléné (60 OE) éther de myristyl glycol.

On remarque que toutes les compositions à l'exception de $B_3$ sont transparentes et ne présentent pas de halo, aussi bien au moment de leur préparation qu'après plusieurs mois de stockage. L'exemple $B_3$ ne fait pas partie de l'invention.

**Tableau 1**

| | $B_1$ | $B_2$ | $B_3$ | $B_4$ | $B_5$ | $B_6$ | $B_7$ | $B_8$ |
|---|---|---|---|---|---|---|---|---|
| **Epaississant** | $E_1$/1% $E_2$/0,9% $E_{10}$/3,5% | $E_1$/0,75% $E_2$/0,9% $E_{10}$/3,5% | $E_2$/0;9% $E_{10}$/2,1% $E_{11}$/0,5% | $E_1$/1,3% $E_2$/0,9% $E_{10}$/2,1 % | $E_1$/1% $E_2$/0,9% $E_{10}$/2,1% | $E_1$/1,5% /$E_2$/0,9% $E_{10}$/2,1% | $E_1$/2% $E_2$/0,9% $E_{10}$/2,1% | $E_1$/1% $E_2$/0,9% $E_{10}$/2,1% |
| Emulsionnant Tween 20 | 4 % | 5 % | 6 % | 10 % | 11 % | 6 % | 6 % | 6 % |
| Miranol C2M | 1,9 % | 1,9 % | 1,9 % | 1,9 % | 1,9 % | 1,9 % | -- | 3,8 % |
| Plantareen 2000 | 2,5 % | 2,5 % | 2,5 % | 2,5 % | -- | 2,5 % | 2,65 % | 2,65 % |
| Glycérol | 4 | 4 | 0 | 4 | 4 | 4 | 4 | 4 |
| Acide citrique | 2 | 2 | 2 | 2 | 2 | 2 | 2 | 2 |
| Phénoxy-2 éthanol | 1 | 1 | 1 | 1 | 1 | 1 | 1 | 1 |
| parfum | -- | 1 | 0,8 | 0,8 | 0,8 | -- | 0,6 | 0,6 |
| Eau de Lucas | -- | -- | -- | 5 | -- | -- | -- | -- |
| Eau | qsp 100 | qsp 100 | qsp 100 | qsp 100 | qsp 100 | qsp 100 | qsp 100 | qsp 100 |
| Viscosité | 66 - 70 | 90 - 96 | 16 - 22 | 50 - 51 | 14 - 20 | 88 - 94 | 72 - 78 | 36 - 40 |
| Transparence | + | + | 0 | + | + | + | + | + |
| Stabilité | 1 | 1 | 0 | 1 | 1 | 1 | 1 | 1 |

| | $B_9$ | $B_{10}$ | $B_{11}$ | $B_{12}$ | $B_{13}$ | $B_{14}$ | $B_{15}$ | $B_{16}$ |
|---|---|---|---|---|---|---|---|---|
| **Epaississant** | $E_5$/0,5% $E_2$/0,9% $E_{10}$/2,1% | $E_2$/0,9% $E_6$/0,5% $E_{10}$/2,1 % | $E_7$/ 0,77% $E_2$/0,9% $E_{10}$/2,1 % | $E_2$/0,9% $E_8$/0,5% $E_{10}$/2,1 % | $E_2$/0,9% $E_9$/1% $E_{10}$/2,1 % | $E_2$/0,9% $E_3$/1% $E_{10}$/2,1 % | $E_2$/0,9% $E_4$/0,5% $E_{10}$/2,1 % | $E_2$/1,8% $E_{10}$/2,1 % |
| Tween 20 | 6% | 6% | 6% | 6% | 6% | 6% | 6% | 6% |
| Miranol | 1,9 % | 1,9 % | 1,9 % | 1,9 % | 1,9 % | 1,9 % | 1 ,9 % | 1,9 % |
| Plantareen | 2,5 % | 2,65 °% | 2,65 % | 2,65 % | 2,65 % | 2,65 % | 2,65 % | 2,65 % |
| Glycérol | -- | -- | -- | -- | -- | -- | -- | -- |
| Acide citrique | 2 % | 2 % | 2 % | 2 % | 2 % | 2 % | 2 % | 2 % |
| Phénoxy-2 éthanol | 1% | 1% | 1 % | 1% | 1 % | 1 % | 1 % | 1 % |
| Parfum | 0,8 % | 0,8 % | 0,8 % | 0,8 % | 0,8 % | 0,8 % | 0,8 % | 0,8 % |
| Eau de lucas | -- | -- | -- | -- | -- | -- | -- | -- |
| Eau | qsp 100 | qsp 100 | qsp 100 | qsp 100 | qsp 100 | qsp 100 | qsp 100 | qsp 100 |
| Viscosité | 20 -26 | 14 - 22 | 16 - 20 | 10 - 18 | 18 - 22 | 12 - 20 | 14 - 18 | 6 -12 |
| Transparence | + | + | + | + | + | + | + | + |
| Stabilité | 1 | 1 | 1 | 1 | 1 | 1 | 1 | 1 |

## Revendications

**1.** Dispositif pressurisé comprenant

(i) une bouteille pressurisée munie d'une tête de distribution,
(ii) une composition transparente monophasique constituée par

A- 0,5 à 10% en poids d'un gaz propulseur, choisi parmi les gaz hydrocarbonés ou fluorés
B- 90 à 99,5% en poids d'une composition aqueuse, exempte d'alcools monohydrique en $C_1$-$C_3$

ce dispositif étant apte à distribuer une mousse lorsque l'on actionne la tête de distribution, la composition B comprend un mélange de tensioactifs choisis parmi les tensioactifs non-ioniques et comprenant éventuellement un ou plusieurs tensioactifs amphotères, en l'absence de tensioactif anioniques et cationiques.

**2.** Dispositif selon la revendication 1, **caractérisé en ce que** la bouteille pressurisé est transparente.

**3.** Dispositif selon l'une quelconque des revendications précédentes, **caractérisé en ce que** la composition aqueuse B comprend :

(a) au moins un tensioactif émulsionnant non-ionique,
(b) au moins un tensioactif épaississant non-ionique.

**4.** Dispositif selon l'une quelconque des revendications précédentes, **caractérisé en ce que** la composition aqueuse B comprend 1 à 25% d'au moins un tensioactif émulsionnant non-ionique.

**5.** Dispositif selon l'une quelconque des revendications précédentes, **caractérisé en ce que** la composition aqueuse B comprend 1 à 30% d'au moins un tensioactif épaississant non-ionique.

**6.** Dispositif selon l'une quelconque des revendications précédentes, **caractérisé en ce que** la composition B a une viscosité Brookfield comprise entre 5 et 200mPa.s.

**7.** Dispositif selon l'une quelconque des revendications précédentes, **caractérisé en ce qu'**il comprend de 1,5 à 4% de gaz propulseur.

**8.** Dispositif selon l'une quelconque des revendications précédentes, **caractérisé en ce que** le gaz est choisi parmi les gaz hydrocarbonés ayant 2 à 6 atomes de carbone.

**9.** Dispositif selon l'une quelconque des revendications 3 à 8, **caractérisé en ce que** le tensio-actif émulsionnant est choisi parmi ceux ayant une balance HLB comprise entre 12 et 18.

**10.** Dispositif selon l'une quelconque des revendications 3 à 9, **caractérisé en ce que** le tensio-actif émulsionnant est choisi parmi les produits d'addition de 1 à 200 moles d'oxyde d'éthylène ou d'oxyde de propylène sur des esters partiels de polyols ayant 2 à 16 atomes de carbone et d'acides gras ayant 12 à 22 atomes de carbone.

**11.** Dispositif selon la revendications 10, **caractérisé en ce que** le tensio-actif émulsionnant a un taux d'estérification inférieur ou égal à 2.

**12.** Dispositif selon l'une quelconque des revendications 3 à 11, **caractérisé en ce que** le tensio-actif épaississant est choisi parmi :

$\alpha$- les $C_1$-$C_6$ alcanolamides d'acide $C_8$-$C_{22}$ alkyl éther carboxylique ;
$\beta$- les produits d'addition de 10 à 300 moles d'oxyde d'éthylène ou d'oxyde de propylène sur des esters partiels de polyols ayant 2 à 16 atomes de carbone et d'acides gras ayant 12 à 22 atomes de carbone.
$\gamma$- les alcools gras en $C_{12}$-$C_{22}$ polyoxyéthylénés et/ou polyoxypropylénés et/ou polyglycérolès,
$\delta$- les esters gras en $C_{12}$-$C_{22}$ de polyoxyéthylène et/ou de polyoxypropylène et/ou de polyglycérol,
$\varepsilon$- les polymères blocs de polyéthylène glycol et/ou de polypropylène glycol.

**13.** Dispositif selon la revendication 12, **caractérisé en ce que** la composition aqueuse B comprend au moins deux tensioactifs épaississants appartenant chacun à deux familles différentes, choisies parmi les familles d'épaississants $\alpha$ à c.

**14.** Dispositif selon l'une quelconque des revendications 10 à 13, **caractérisé en ce que** les polyols sont choisis parmi

le glycol, le glycérol, le glucose, le fructose, le maltose, le ribose, l'arabinose, le xylose, le lyxose, le ribulose, le xylulose, l'allose, l'altrose, le mannose, le gulose, le galactose, le saccharose, le sorbitol.

**15.** Dispositif selon l'une quelconque des revendications 3 à 14, **caractérisé en ce que** le tensioactif épaississant est choisi parmi l'huile de ricin, les monoéthanotamides d'acide alkyl ($C_{13}$-$C_{15}$) éther carboxylique (2 OE), la monoéthanolamide d'acide éther carboxylique (50 OE), les mono- et di-glycérides de suif oxyéthylénés (200 OE), les mono- et di-glycérides de suif oxyéthylénés (82 OE), les mono- et di-glycérides de coprah oxyéthylénés (78 OE), les triglycérides d'acide stéarique et hydroxystéarique (40 OE), le mono-stéarate de polyéthylène glycol (100 OE), l'alcool dodécanediol polyglycérolé (3,5moles), l'octyl-2 dodécanol oxyéthyléné (250 OE), l'alcool stéarylique oxyéthyléné (100 OE), l'alcool oléique oxyéthyléné (50 OE), le di-oléate de polyéthylène glycol (50 OE), le palmitate de glycérol oxyéthyléné (200 OE), le tri-isostéarate de sorbitane oxyéthyléné (160 OE), le tri-isostéarate de sorbitane oxyéthyléné (200 OE), le stéarate de polyéthylèneglycol (150 OE),), le di-oléate de méthyl glucose oxyéthyléné (120 OE), le tètra-stèarate de penta-érythrityle oxyèthyléné (150 OE), le di-oléate de polyéthylène glycol (55 OE), l'undécylénate de polyéthylène glycol (300 OE), le di-stéarate de polyéthylène glycol (150 OE).

**16.** Dispositif selon l'une quelconque des revendications 3 à 15, **caractérisé en ce que** le tensioactif émulsionnant est choisi parmi le mono-undécylénate de glycérol oxyéthyléné (7 OE), le monostéarate de glycérol oxyéthyléné (15 OE), le mono-laurate de glycérol oxyéthyléné (20 OE), le di-laurate de glycérol oxyéthyléné (20 OE), le stéarate de di-glycérol oxyéthyléné (4 OE), le cocoate de glycérol oxyéthyléné (7 OE), le pyrrolidone carboxylate de monoisostéarate de glycérol oxyéthyléné (25OE), le mono stéarate de glycérol oxyéthyléné (20 OE), le mono-stéarate de glycérol oxyéthyléné (30 OE), le stéarate de di-glycérol oxyéthyléné (4 OE), les mono- et di-glycérides de coprah oxyéthyléné (30 OE), le lanolate de sorbitane oxyéthyléné (40 OE), le mono-oléate de sorbitane oxyéthyléné (20 OE), le tri-oléate de sorbitane oxyéthyléné (20 OE), le mono-palmitale de sorbitane oxyéthyléné (20 OE), le monostéarate de sorbitane oxyéthyléné (20 OE), le mono-laurate de sorbitane oxyéthyléné (20 OE), l'undécylénate de sorbitane oxyéthyléné (20 OE), l'undécylénate de sorbitane oxyéthyléné (18 OE), l'hexastéarate de sorbitane oxyéthyléné (6 OE), le mono-laurate de sorbitane oxyéthyléné (44 OE), le tétra-oléate de sorbitane oxyéthyléné (30 OE), le mono-stéarate de sorbitane oxyéthyléné (4 OE), l'oléate de sorbitane oxyéthyléné (40 OE), le mono-laurate de sorbitane oxyéthyléné (4 OE), le mono-laurate de sorbitane oxyéthyléné (10 OE), le tétra-oléate de sorbitane oxyéthyléné (40 OE), le cocoate de butyl glucoside oxyéthyléné (3 OE), le di-stéarate de méthyl glucoside oxypropyléné (20 OP), le mono-laurate de méthyl glucoside oxyéthyléné (20 OE), le benzoate de méthyl glucose oxyéthyléné (20 OE), les esters de tri-glycérides de ricin et de saccharose oxyéthylénés (1,4 OE), les esters de triglycérides de ricin et de saccharose oxyéthylénés (2 OE), le sesquistéarate de méthyl glucose oxyéthyléné (20 OE), le di-stéarate de méthyl glucose oxyéthyléné (20 OE), le mono-stéarate de butanediol oxyéthyléné (4 OE), le mono-stéarate de polyéthylène glycol oxyéthyléné (50 OE), le di-stéarate de polyéthylène glycol, le myristate de polyéthylène glycol (8 OE), le mono-stéarate de polyéthylène glycol (8 OE), le mono-stéarate de polyéthylène glycol (20 OE), le laurate de polyéthylène glycol (6 OE), le di-stéarate de polyéthylène glycol (8 OE), le di-laurate de polyéthylène glycol (8 OE), le mono-oléate de polyéthylène glycol (8 OE), le di-heptanoate de tétra-éthyléne glycol, le di-oléate de polyéthylène glycol (8 OE), l'oléate de polypropylène glycol (26 OE), le di-polyhydroxystéarate (6 hydroxy) de polyéthylène glycol (30 OE), la stéarate de polyéthylène glycol (40 OE), le di-laurate de polyéthylène glycol (8 OE).

**17.** Dispositif selon l'une quelconque des revendications précédentes, **caractérisé en ce que** la composition aqueuse B comprend :

(a) 2 à 15% en poids d'au moins un tensioactif émulsionnant non-ionique.
(b) 2 à 15% en poids d'au moins un tensioactif épaississant non-ionique.

**18.** Dispositif selon l'une quelconque des revendications précédentes, **caractérisé en ce que** la composition aqueuse B comprend 3 à 12% en poids d'au moins un tensioactif émulsionnant non-ionique.

**19.** Dispositif selon l'une quelconque des revendications précédentes, **caractérisé en ce que** la composition aqueuse B a une viscosité Brookfield comprise entre 10 et 150mPa.s.

**20.** Dispositif selon l'une quelconque des revendications précédentes, **caractérisé en ce que** la composition aqueuse B comprend de 3 à 10% en poids d'au moins un tensio-actif épaississant.

**21.** Dispositif selon l'une quelconque des revendications précédentes, **caractérisé en ce que** la composition aqueuse B comprend au moins un alkylpolyglucoside comme tensio-actif non-ionique.

**22.** Dispositif selon l'une quelconque des revendications précédentes, **caractérisé en ce que** la composition aqueuse B comprend 0,01 à 10% en poids d'au moins un alkylpolyglucoside.

**23.** Dispositif selon l'une quelconque des revendications précédentes, **caractérisé en ce que** la composition aqueuse B comprend au moins un tensioactif amphotère.

**24.** Dispositif selon la revendication 23, **caractérisé en ce que** le tensioactif amphotère est présent dans la composition aqueuse B en quantités allant de 0,01 à 15% en poids.

**25.** Dispositif selon l'une quelconque des revendications précédentes, **caractérisé en ce que** B comprend :

- 1 à 25% en poids d'au moins un tensioactif émulsionnant non-ionique,
- 1 à 30% en poids d'au moins un tensioactif épaississant non-ionique,
- 0.01 à 10% en poids d'au moins un alkylpolyglucoside,
- 0,01 à 15% en poids d'au moins un tensioactif amphotère.

**26.** Dispositif selon l'une quelconque des revendications précédentes, **caractérisé en ce que** la composition comprend une eau minérale ou une eau thermale.

**27.** Dispositif selon l'une quelconque des revendications précédentes, **caractérisé en ce que** la composition comprend en outre au moins un adjuvant choisi parmi: les conservateurs, les antioxydants, les parfums, les filtres, les matières colorantes, les actifs hydrophiles ou lipophiles.

**28.** Dispositif selon l'une quelconque des revendications précédentes, **caractérisé en ce que** la composition comprend en outre au moins un $\alpha$-hydroxy-acide.

**29.** Dispositif selon l'une quelconque des revendications précédentes, **caractérisé en ce que** la composition comprend en outre au moins un anti-oxydant choisi parmi les sulfites.

**30.** Dispositif selon l'une quelconque des revendications précédentes, **caractérisé en ce que** la composition est adaptée au nettoyage et au soin de la peau humaine.

## Claims

**1.** Pressurized device including

(i) a pressurized bottle provided with a dispensing head,
(ii) a single-phase transparent composition constituted of

A- 0.5 to 10 % by weight of a propellent gas chosen from hydrocarbon or fluorinated gases,
B- 90 to 99.5 % by weight of an aqueous composition which is free from $C_1$-$C_3$ monohydric alcohols,

this device being suitable for dispensing a foam when the dispensing head is actuated, the composition B including a mixture of surfactants chosen from nonionic surfactants and optionally including one or more amphoteric surfactants, in the absence of anionic and cationic surfactants.

**2.** Device according to Claim 1, **characterized in that** the pressurized bottle is transparent.

**3.** Device according to either of the preceding claims, **characterized in that** the aqueous composition B includes:

(a) at least one nonionic emulsifying surfactant,
(b) at least one nonionic thickening surfactant.

**4.** Device according to any one of the preceding claims, **characterized in that** the aqueous composition B includes 1 to 25 % of at least one nonionic emulsifying surfactant.

**5.** Device according to any one of the preceding claims, **characterized in that** the aqueous composition B includes

1 to 30 % of at least one nonionic thickening surfactant.

6. Device according to any one of the preceding claims, **characterized in that** the composition B has a Brookfield viscosity of between 5 and 200 mPa.s.

7. Device according to any one of the preceding claims, **characterized in that** it includes from 1.5 to 4 % of propellent gas.

8. Device according to any one of the preceding claims, **characterized in that** the gas is chosen from hydrocarbon gases containing 2 to 6 carbon atoms.

9. Device according to any one of Claims 3 to 8, **characterized in that** the emulsifying surfactant is chosen from those which have an HLB balance of between 12 and 18.

10. Device according to any one of Claims 3 to 9, **characterized in that** the emulsifying surfactant is chosen from the products of addition of I to 200 mol of ethylene oxide or of propylene oxide to partial esters of polyols containing 2 to 16 carbon atoms and of fatty acids containing 12 to 22 carbon atoms.

11. Device according to Claim 10, **characterized in that** the emulsifying surfactant has a degree of esterification lower than or equal to 2.

12. Device according to any one of Claims 3 to 11, **characterized in that** the thickening surfactant is chosen from:

α- the $C_1$-$C_6$ alkanolamides of carboxylic $C_8$-$C_{22}$ alkyl ether acid,
β- the products of addition of 10 to 300 mol of ethylene oxide or of propylene oxide to partial esters of polyols containing 2 to 16 carbon atoms and of fatty acids containing 12 to 22 carbon atoms,
γ- polyoxyethylenated and/or polyoxypropylenated and/or polyglycerolated $C_{12}$-$C_{22}$ fatty alcohols,
δ- $C_{12}$-$C_{22}$ fatty esters of polyoxyethylene and/or of polyoxypropylene and/or of polyglycerol,
ε- polyethylene glycol and/or polypropylene glycol block polymers.

13. Device according to Claim 12, **characterized in that** the aqueous composition B includes at least two thickening surfactants, each belonging to two different classes which are chosen from the thickener classes α to ε.

14. Device according to any one of Claims 10 to 13, **characterized in that** the polyols are chosen from glycol, glycerol, glucose, fructose, maltose, ribose, arabinose, xylose, lyxose, ribulose, xylulose, allose, altrose, mannose, gulose, galactose, sucrose and sorbitol.

15. Device according to any one of Claims 3 to 14, **characterized in that** the thickening surfactant is chosen from castor oil, monoethanolamides of carboxylic alkyl ($C_{13}$, $C_{15}$) ether acid (2 EO), the monoethanolamide of carboxylic ether acid (50 EO), oxyethylenated (200 EO) tallow mono- and diglycerides, oxyethylenated (82 EO) tallow mono- and diglycerides, oxyethylenated (78 EO) coconut mono- and diglycerides, stearic and hydroxystearic acid triglycerides (40 EO), polyethylene glycol monostearate (100 EO), polyglycerolated (3.5 mol) dodecanediol alcohol, oxyethylenated (250 EO) 2-octyldodecanol, oxyethylenated (100 EO) stearyl alcohol, oxyethylenated (50 EO) oleyl alcohol, polyethylene glycol (50 EO) dioleate, oxyethylenated (200 EO) glyceryl palmitate, oxyethylenated (160 EO) sorbitan triisostearate, oxyethylenated (200 EO) sorbitan triisostearate, polyethylene glycol (150 EO) stearate, oxyethylenated (120 EO) methylglucose dioleate, oxyethylenated (150 EO) pentaerythrityl tetrastearate, polyethylene glycol (55 EO) dioleate, polyethylene glycol (300 EO) undecylenate and polyethylene glycol (150 EO) distearate.

16. Device according to any one of Claims 3 to 15, **characterized in that** the emulsifying surfactant is chosen from oxyethylenated (7 EO) glyceryl monoundecylenate, oxyethylenated (15 EO) glyceryl monostearate, oxyethylenated (20 EO) glyceryl monolaurate, oxyethylenated (20 EO) glyceryl dilaurate, oxyethylenated (4 EO) diglyceryl stearate, oxyethylenated (7 EO) glyceryl cocoate, the pyrrolidonecarboxylate of oxyethylenated (25 EO) glyceryl monoisostearate, oxyethylenated (20 EO) glyceryl monostearate, oxyethylenated (30 EO) glyceryl monostearate, oxyethylenated (4 EO) diglyceryl stearate, oxyethylenated (30 EO) coconut mono- and diglycerides, oxyethylenated (40 EO) sorbitan lanolate, oxyethylenated (20 EO) sorbitan monooleate, oxyethylenated (20 EO) sorbitan trioleate, oxyethylenated (20 EO) sorbitan monopalmitate, oxyethylenated (20 EO) sorbitan monostearate, oxyethylenated (20 EO) sorbitan monolaurate, oxyethylenated (20 EO) sorbitan undecylenate, oxyethylenated (18 EO) sorbitan undecylenate, oxyethylenated (6 EO) sorbitan hexastearate, oxyethylenated (44 EO) sorbitan monolaurate, oxyethylenated (30

EO) sorbitan tetraoleate, oxyethylenated (4 EO) sorbitan monostearate, oxyethylenated (40 EO) sorbitan oleate, oxyethylenated (4 EO) sorbitan monolaurate, oxyethylenated (10 EO) sorbitan monolaurate, oxyethylenated (40 EO) sorbitan tetraoleate, oxyethylenated (3 EO) butylglucoside cocoate, oxypropylenated (20 PO) methylglucoside distearate, oxyethylenated (20 EO) methylglucoside monolaurate, oxyethylenated (20 EO) methylglucose benzoate, oxyethylenated (1.4 EO) castor and sucrose triglyceride esters, oxyethylenated (2 EO) castor and sucrose triglyceride esters, oxyethylenated (20 EO) methylglucose sesquistearate, oxyethylenated (20 EO) methylglucose distearate, oxyethylenated (4 EO) butanediol monostearate, oxyethylenated (50 EO) polyethylene glycol monostearate, polyethylene glycol distearate, polyethylene glycol (8 EO) myristate, polyethylene glycol (8 EO) monostearate, polyethylene glycol (20 EO) monostearate, polyethylene glycol (6 EO) laurate, polyethylene glycol (8 EO) distearate, polyethylene glycol (8 EO) dilaurate, polyethylene glycol (8 EO) monoolcate, tetraethylene glycol diheptanoate, polyethylene glycol (8 EO) dioleate, polypropylene glycol (26 EO) oleate, polyethylene glycol (30 EO) dipolyhydroxystearate (6 hydroxy), polyethylene glycol (40 EO) stearate and polyethylene glycol (8 EO) dilaurate.

**17.** Device according to any one of the preceding claims, **characterized in that** the aqueous composition B includes:

(a) 2 to 15 % by weight of at least one nonionic emulsifying surfactant,
(b) 2 to 15 % by weight of at least one nonionic thickening surfactant.

**18.** Device according to any one of the preceding claims, **characterized in that** the aqueous composition B includes 3 to 12 % by weight of at least one nonionic emulsifying surfactant.

**19.** Device according to any one of the preceding claims, **characterized in that** the aqueous composition B has a Brookfield viscosity of between 10 and 150 mPa.s.

**20.** Device according to any one of the preceding claims, **characterized in that** the aqueous composition B includes from 3 to 10 % by weight of at least one thickening surfactant.

**21.** Device according to any one of the preceding claims, **characterized in that** the aqueous composition B includes at least one alkylpolyglucoside as nonionic surfactant.

**22.** Device according to any one of the preceding claims, **characterized in that** the aqueous composition B includes 0.01 to 10 % by weight of at least one alkylpolyglucoside.

**23.** Device according to any one of the preceding claims, **characterized in that** the aqueous composition B includes at least one amphoteric surfactant.

**24.** Device according to Claim 23, **characterized in that** the amphoteric surfactant is present in the aqueous composition B in quantities ranging from 0.01 to 15 % by weight.

**25.** Device according to any one of the preceding claims, **characterized in that** B includes:

- 1 to 25 % by weight of at least one nonionic emulsifying surfactant,
- 1 to 30 % by weight of at least one nonionic thickening surfactant,
- 0.01 to 10 % by weight of at least one alkylpolyglucoside,
- 0.01 to 15 % by weight of at least one amphoteric surfactant.

**26.** Device according to any one of the preceding claims, **characterized in that** the composition includes a mineral water or a thermal water.

**27.** Device according to any one of the preceding claims, **characterized in that** the composition additionally includes at least one adjuvant chosen from preservatives, antioxidants, perfumes, screening agents, colorants and hydrophilic or lipophilic agents.

**28.** Device according to any one of the preceding claims, **characterized in that** the composition additionally includes at least one α-hydroxy acid.

**29.** Device according to any one of the preceding claims, **characterized in that** the composition additionally includes at least one antioxidant chosen from sulphites.

**30.** Device according to any one of the preceding claims, **characterized in that** the composition is suitable for cleansing and for the care of human skin.

## Patentansprüche

**1.** Druckgasvorrichtung, enthaltend

(i) eine Druckgasflasche, die mit einem Verteilerkopf ausgestattet ist, und
(ii) eine durchsichtige einphasige Zusammensetzung, gebildet aus

A -0,5 bis 10 Gew.-% eines Treibgases, das unter den Kohlenwasserstoffgasen oder fluorierten Gasen ausgewählt ist, und
B -90 bis 99,5 Gew.-% einer wässerigen Zusammensetzung, die keine einwertigen Alkohole mit 1 bis 3 Kohlenstoffatomen enthält,

wobei diese Vorrichtung befähigt ist, bei Betätigung des Verteilerkopfes einen Schaum abzugeben, die Zusammensetzung B ein Gemisch von grenzflächenaktiven Stoffen enthält, die unter den nichtionischen grenzflächenaktiven Stoffen ausgewählt sind, wobei gegebenenfalls ein oder mehrere amphotere grenzflächenaktive Stoffe und keine anionischen und kationischen grenzflächenaktiven Stoffe enthalten sind.

**2.** Vorrichtung nach Anspruch 1, **dadurch gekennzeichnet, dass** die Druckgasflasche durchsichtig ist.

**3.** Vorrichtung nach einem der vorhergehenden Ansprüche, **dadurch gekennzeichnet, dass** die wässerige Zusammensetzung B enthält:

(a) mindestens einen emulgierenden nichtionischen grenzflächenaktiven Stoff und
(b) mindestens einen verdickenden nichtionischen grenzflächenaktiven Stoff.

**4.** Vorrichtung nach einem der vorhergehenden Ansprüche, **dadurch gekennzeichnet, dass** die wässerige Zusammensetzung B 1 bis 25 % mindestens eines emulgierenden nichtionischen grenzflächenaktiven Stoffes enthält.

**5.** Vorrichtung nach einem der vorhergehenden Ansprüche, **dadurch gekennzeichnet, dass** die wässerige Zusammensetzung B 1 bis 30 % mindestens eines verdickenden nichtionischen grenzflächenaktiven Stoffes enthält.

**6.** Vorrichtung nach einem der vorhergehenden Ansprüche, **dadurch gekennzeichnet, dass** die Zusammensetzung B eine Brookfield-Viskosität im Bereich von 5 bis 200 mPas aufweist.

**7.** Vorrichtung nach einem der vorhergehenden Ansprüche, **dadurch gekennzeichnet, dass** sie 1,5 bis 4 % Treibgas enthält.

**8.** Vorrichtung nach einem der vorhergehenden Ansprüche, **dadurch gekennzeichnet, dass** das Gas unter den gasförmigen Kohlenwasserstoffen mit 2 bis 6 Kohlenstoffatomen ausgewählt ist.

**9.** Vorrichtung nach einem der Ansprüche 3 bis 8, **dadurch gekennzeichnet, dass** der emulgierende grenzflächenaktive Stoff unter den grenzflächenaktiven Stoffen ausgewählt ist, die einen HLB-Wert im Bereich von 12 bis 18 aufweisen.

**10.** Vorrichtung nach einem der Ansprüche 3 bis 9, **dadurch gekennzeichnet, dass** der emulgierende grenzflächenaktive Stoff unter den Additionsprodukten von 1 bis 200 Mol Ethylenoxid oder Propylenoxid an partiellen Estern von Polyolen mit 2 bis 16 Kohlenstoffatomen und Fettsäuren mit 12 bis 22 Kohlenstoffatomen ausgewählt ist.

**11.** Vorrichtung nach Anspruch 10, **dadurch gekennzeichnet, dass** der emulgierende grenzflächenaktive Stoff einen Veresterungsgrad von 2 oder darunter aufweist.

**12.** Vorrichtung nach einem der Ansprüche 3 bis 11, **dadurch gekennzeichnet, dass** der verdickende grenzflächenaktive Stoff ausgewählt ist unter:

α- den $C_{1-6}$-Alkanolamiden einer $C_{8-22}$-Alkylethercarbonsäure,
β- den Additionsprodukten von 10 bis 300 Mol Ethylenoxid oder Propylenoxid an partiellen Estern von Polyolen mit 2 bis 16 Kohlenstoffatomen und Fettsäuren mit 12 bis 22 Kohlenstoffatomen,
γ- den polyethoxylierten und/oder polypropoxylierten und/oder mehrfach mit Glycerin veretherten $C_{12-22}$-Fettalkoholen,
δ- den $C_{12-22}$-Fettsäureestern von Polyoxyethylen und/ oder Polyoxypropylen und/oder Polyglycerin und
ε- den Blockpolymeren von Polyethylenglycol und/oder Polypropylenglycol.

**13.** Vorrichtung nach Anspruch 12, **dadurch gekennzeichnet, dass** die wässerige Zusammensetzung B mindestens zwei verdickende grenzflächenaktive Stoffe aufweist, die jeweils zwei unterschiedlichen Gruppen, die unter den Verdickungsmittelgruppen α bis ε ausgewählt sind, angehören.

**14.** Vorrichtung nach einem der Ansprüche 10 bis 13, **dadurch gekennzeichnet, dass** die Polyole unter Glycol, Glycerin, Glucose, Fructose, Maltose, Ribose, Arabinose, Xylose, Lyxose, Ribulose, Xylulose, Allose, Altrose, Mannose, Gulose, Galactose, Saccharose und Sorbit ausgewählt sind.

**15.** Vorrichtung nach einem der Ansprüche 3 bis 14, **dadurch gekennzeichnet, dass** der verdickende grenzflächenaktive Stoff unter Ricinusöl, Monoethanolamiden einer Alkyl($C_{13,15}$)ethercarbonsäure (2 EO), dem Monoethanolamid einer Ethercarbonsäure (50 EO), ethoxylierten Mono- und Diglyceriden von Talg (200 EO), ethoxylierten Mono- und Diglyceriden von Talg (82 EO), ethoxylierten Mono- und Diglyceriden von Kopra (78 EO), Triglyceriden von Stearinsäure und Hydroxystearinsäure (40 EO), Polyethylenglycolmonostearat (100 EO), mit Glycerin verethertem Dodecandiol (3,5 Mol), ethoxyliertem 2-Octyldodecanol (250 EO), ethoxyliertem Stearylalkohol (100 EO), ethoxyliertem Oleylalkohol (50 EO), Polyethylenglycoldioleat (50 EO), ethoxyliertem Glycerinpalmitat (200 EO), ethoxyliertem Sorbitantriisostearat (160 EO), ethoxyliertem Sorbitantriisostearat (200 EO), Polyethylenglycolstearat (150 EO), ethoxyliertem Methylglucosedioleat (120 EO), ethoxyliertem Pentaerythrityltetrastearat (150 EO), Polyethylenglycoldioleat (55 EO), Polyethylenglycolundecylenat (300 EO) und Polyethylenglycoldistearat (150 EO) ausgewählt ist.

**16.** Vorrichtung nach einem der Ansprüche 3 bis 15, **dadurch gekennzeichnet, dass** der emulgierende grenzflächenaktive Stoff unter ethoxyliertem Glycerinmonoundecylenat (7 EO), ethoxyliertem Glycerinmonostearat (15 EO), ethoxyliertem Glycerinmonolaurat (20 EO), ethoxyliertem Glycerindilaurat (20 EO), ethoxyliertem Diglycerinstearat (4 EO), ethoxyliertem Glycerincocoat (7 EO), ethoxyliertem Pyrrolidoncarboxylat von Glycerinmonoisostearat (25 EO), ethoxyliertem Glycerinmonostearat (20 EO), ethoxyliertem Glycerinmonostearat (30 EO), ethoxyliertem Diglycerinstearat (4 EO), ethoxylierten Mono-und Diglyceriden von Kopra (30 EO), ethoxyliertem Sorbitanlanolat (40 EO), ethoxyliertem Sorbitanmonooleat (20 EO), ethoxyliertem Sorbitantrioleat (20 EO), ethoxyliertem Sorbitanmonopalmitat (20 EO), ethoxyliertem Sorbitanmonostearat (20 EO), ethoxyliertem Sorbitanmonolaurat (20 EO), ethoxyliertem Sorbitanundecylenat (20 EO), ethoxyliertem Sorbitanundecylenat (18 EO), ethoxyliertem Sorbitanhexastearat (6 EO), ethoxyliertem Sorbitanmonolaurat (44 EO), ethoxyliertem Sorbitantetraoleat (30 EO), ethoxyliertem Sorbitanmonostearat (4 EO), ethoxyliertem Sorbitanoleat (40 EO), ethoxyliertem Sorbitanmonolaurat (4 EO), ethoxyliertem Sorbitanmonolaurat (10 EO), ethoxyliertem Sorbitantetraoleat (40 EO), ethoxyliertem Butylglucosidcocoat (3 EO), propoxyliertern Methylglucosiddistearat (20 PO), ethoxyliertem Methylglucosidmonolaurat (20 EO), ethoxyliertem Methylglucosebenzoat (20 EO), ethoxylierten Estern von Triglyceriden von Ricinusöl und Saccharose (1,4 EO), ethoxylierten Estern von Triglyceriden von Ricinusöl und Saccharose (2 EO), ethoxyliertem Methylglucosesesquistearat (20 EO), ethoxyliertem Methylglucosedistearat (20 EO), ethoxyliertem Butandiolmonostearat (4 EO), ethoxyliertem Polyethylenglycolmonostearat (50 EO), Polyethylenglycoldistearat, Polyethylenglycolmyristat (8 EO), Polyethylenglycolmonostearat (8 EO), Polyethylenglycolmonostearat (20 EO), Polyethylenglycollaurat (6 EO), Polyethylenglycoldistearat (8 EO), Polyethylenglycoldilaurat (8 EO), Polyethylenglycolmonooleat (8 EO), Tetraethylenglycoldiheptanoat, Polyethylenglycoldioleat (8 EO), Polypropylenglycololeat (26 EO), Dipolyhydroxystearat-(6-hydroxy) von Polyethylenglycol (30 EO), Polyethylenglycolstearat (40 EO) und Polyethylenglycoldilaurat (8 EO) ausgewählt ist.

**17.** Vorrichtung nach einem der vorhergehenden Ansprüche, **dadurch gekennzeichnet, dass** die wässerige Zusammensetzung B

(a) 2 bis 15 Gew.-% mindestens eines emulgierenden nichtionischen grenzflächenaktiven Stoffes und
(b) 2 bis 15 Gew.-% mindestens eines verdickenden nichtionischen grenzflächenaktiven Stoffes enthält.

**18.** Vorrichtung nach einem der vorhergehenden Ansprüche, **dadurch gekennzeichnet, dass** die wässerige Zusammensetzung B 3 bis 12 Gew.% mindestens eines emulgierenden nichtionischen grenzflächenaktiven Stoffes enthält.

**19.** Vorrichtung nach einem der vorhergehenden Ansprüche, **dadurch gekennzeichnet, dass** die wässerige Zusammensetzung B eine Brookfield-Viskosität im Bereich von 10 bis 150 mPa s aufweist.

**20.** Vorrichtung nach einem der vorhergehenden Ansprüche, **dadurch gekennzeichnet, dass** die wässerige Zusammensetzung B 3 bis 10 Ges.% mindestens eines verdickenden grenzflächenaktiven Stoffes enthält.

**21.** Vorrichtung nach einem der vorhergehenden Ansprüche, **dadurch gekennzeichnet, dass** die wässerige Zusammensetzung B mindestens ein Alkylpolyglucosid als nichtionischen grenzflächenaktiven Stoff enthält.

**22.** Vorrichtung nach einem der vorhergehenden Ansprüche, **dadurch gekennzeichnet, dass** die wässerige Zusammensetzung B 0,01 bis 10 Gew.-% mindestens eines Alkylpolyglucosids enthält.

**23.** Vorrichtung nach einem der vorhergehenden Ansprüche, **dadurch gekennzeichnet, dass** die wässerige Zusammensetzung B mindestens einen amphoteren grenzflächenaktiven Stoff enthält.

**24.** Vorrichtung nach Anspruch 23, **dadurch gekennzeichnet, dass** der amphotere grenzflächenaktive Stoff in der wässerigen Zusammensetzung B in Mengenanteilen von 0,01 bis 15 Gew.-% vorliegt.

**25.** Vorrichtung nach einem der vorhergehenden Ansprüche, **dadurch gekennzeichnet, dass** B enthält:

- 1 bis 25 Gew.-% mindestens eines emulgierenden nichtionischen grenzflächenaktiven Stoffes,
- 1 bis 30 Gew.-% mindestens eines verdickenden nichtionischen grenzflächenaktiven Stoffes,
- 0,01 bis 10 Gew.-% mindestens eines Alkylpolyglucosids und
- 0,01 bis 15 Gew.-% mindestens eines amphoteren grenzflächenaktiven Stoffes.

**26.** Vorrichtung nach einem der vorhergehenden Ansprüche, **dadurch gekennzeichnet, dass** die Zusammensetzung ein Mineralwasser oder ein Thermalwasser enthält.

**27.** Vorrichtung nach einem der vorhergehenden Ansprüche, **dadurch gekennzeichnet, dass** die Zusammensetzung ferner mindestens einen Hilfsstoff enthält, der unter den Konservierungsmitteln, Antioxidantien, Parfums, Filtern, Farbmitteln und hydrophilen oder lipophilen Wirkstoffen ausgewählt ist.

**28.** Vorrichtung nach einem der vorhergehenden Ansprüche, **dadurch gekennzeichnet, dass** die Zusammensetzung ferner mindestens eine $\alpha$-Hydroxysäure enthält.

**29.** Vorrichtung nach einem der vorhergehenden Ansprüche, **dadurch gekennzeichnet, dass** die Zusammensetzung ferner mindestens ein Antioxidans, das unter den Sulfiten ausgewählt ist, enthält.

**30.** Vorrichtung nach einem der vorhergehenden Ansprüche, **dadurch gekennzeichnet, dass** die Zusammensetzung für die Reinigung und Pflege der menschlichen Haut angepasst ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 3719752 A
- WO 9505796 A
- EP 676188 A
- CA 1002256
- US 3840465 A
- FR 9512788
- FR 2091516